# EUROPEAN PATENT SPECIFICATION

(11) **EP 0 680 293 B1**
(45) Date of publication and mention of the grant of the patent: **12.05.1999**
(21) Application number: 93909147.6
(22) Date of filing: 24.03.1993
(51) Int. Cl.: A61F 2/66

(54) **ATTACHMENT CONSTRUCTION FOR PROSTHESIS**
BEFESTIGUNGSSYSTEM FÜR PROTHESE
STRUCTURE DE FIXATION DE PROTHESES

(30) Priority: 24.03.1992 US 856666
(43) Date of publication of application: 08.11.1995
(73) Proprietor: PHILLIPS, Van L., Rancho Santa Fe, CA 92067 (US)
(72) Inventor: PHILLIPS, Van L., Rancho Santa Fe, CA 92067 (US)
(74) Representative: Wolhändler, Jacques
(86) International application number: US9302661
(87) International publication number: WO9318723

(56) References cited:
- EP-A- 0 267 347
- DE-A- 3 214 772
- FR-A- 2 119 441
- GB-A- 2 099 950
- US-A- 3 820 169
- US-A- 4 645 509

## Description

This invention relates to prostheses in general, and specifically to a construction which permits the attachment of a prosthetic device having a relatively flat surface to the exterior of a prosthetic pylon which has a generally curvilinear exterior.

It is well known in the prosthetic arts, and particularly in the prosthetic leg art, to provide a cylindrical tubular pylon between a wearer's stump and another prosthetic device, such as a prosthetic foot. Such pylons provide a strong, lightweight extension structure between the stump and other prostheses, which structure may typically be cut to a desirable length for a particular wearer.

Although other pylon configurations could certainly be utilized for this purpose, the cylindrical tubular pylon has numerous desirable characteristics and has therefore become the configuration of choice among may prosthetists and amputees. For example, although prosthetists could utilize solid pylons or pylons having a square or rectangular cross-section or otherwise provide a relatively flat attachment surface for other prosthetic devices, such other pylons typically are not as strong and lightweight as a cylindrical tubular pylon. Moreover, a cylindrical tubular pylon may be relatively less expensive to manufacture than pylons having more complex cross-sectional configurations or requiring more material.

The United States Patent US-A 3,820,169 discloses a lower limb prosthesis including a knee joint to which a foot is attached by means of a tubular pylon. The prosthetic foot comprises a rigid keel having a cylindrical socket arranged to receive the pylon. Flanges are formed on the exterior of the keel through which cap screws are fitted to tighten the socket and clamp the pylon therein.

The PCT application WO93/06795, prior art to be considered under Article 4(3) EPC, discloses an energy-storing prosthetic leg pylon, upper and lower pylon members operatively interconnected by one or more spring elements. The pylon members reciprocate with one another such that forces imposed on the pylon result in energy being stored in the spring elements. Upon removal of the force, the spring elements are capable of removing the stored energy. The lower pylon member may be formed as a cylindrical tubular pylon. The prosthetic foot comprises a flat section which is connected to the tubular pylon by intermediate coupling means.

Other conventional prosthetic devices commonly include relatively flat attachment surfaces for operable attachment to the wearer, whether or not an intermediate pylon is utilized. Thus, in order to be usable with such pylons, these other prosthetic devices must be specifically designed to include special attachment structures and/or configurations which permit attachment to the pylons.

The special designs and modifications of the prior art are not without drawbacks. For example, any such special attachment structure typically adds to the cost of manufacture of the prosthesis. In certain cases, these attachment structures are fairly bulky, weighty, and complex, because the attachment of the aforesaid other prosthetic devices to the pylon must be reliable and secure to prevent undesirable movement between the pylon and other prosthetic device. Perhaps more importantly, the structures and configurations necessary to permit attachment to the pylons may detrimentally affect the performance of the prosthesis and/or limit the range of wearers who could otherwise benefit from its use.

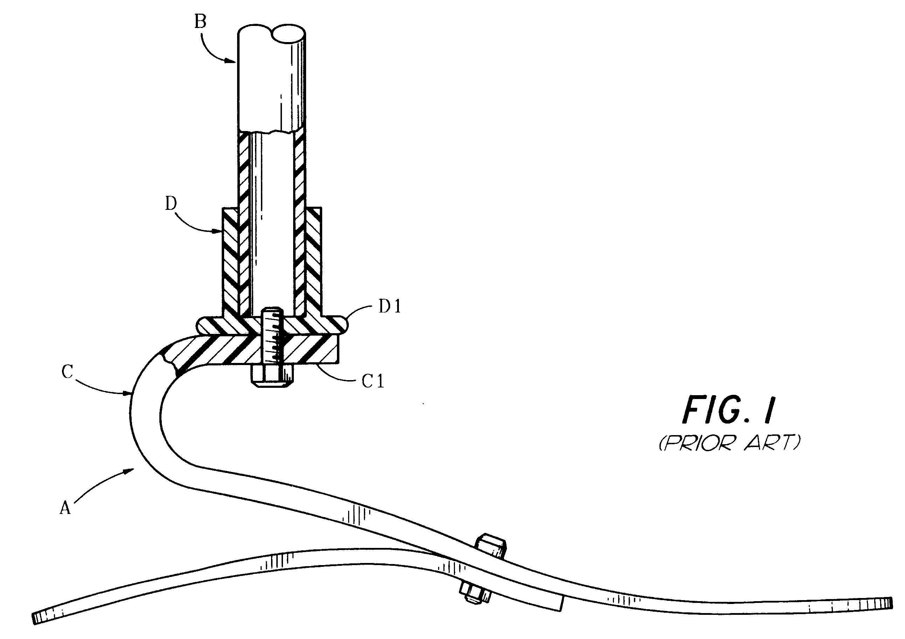

Exemplary of such design accommodations is the configuration illustrated in Fig. 1 hereto, in which a foot prosthesis A is operatively attached to a tubular cylindrical pylon B through the incorporation of a curvilinear section C and the provision of an attachment mechanism D. As shown in Fig. 1, the combination of the curvilinear section C, its attachment section C₁, the attachment mechanism D, and its attachment section D₁ permits the relatively flat structure C₁ to be operatively retained adjacent the relatively flat bottom of the tube B. Such a structure involves a complexity and expense, and includes performance limitations, which are less than optimum.

Moreover, such a structure requires a substantial clearance between the pylon tube B in order to accommodate the components A, C, C₁, D and D₁ thereby limiting the range of amputees who may be served by such a construction.

It is, therefore, an object of my invention to provide a prosthetic construction which will overcome the foregoing problems and, among other things, will permit tubular cylindrical pylons to be readily utilized with other prosthetic devices having otherwise relatively non-curved attachment surfaces. Those skilled in the art will understand, however, that various embodiments of my invention may be practiced with other types of cylindrical pylons; for example, solid pylons, pylons with oval or other curved exterior cross sectional configurations.

The prosthesis of my invention is defined in claim 1.

The construction permits the above-described popular cylindrical tubular pylon to be readily utilized with any of a broad range of other prosthetic devices having attachment surfaces, and permits ready manufacture and ready interchangeability of a range of prosthetic devices and pylons. Economies of manufacture and standardization of parts, ease of assembly and of replacement of prosthetic components, and adjustability of the entire prosthesis are improved and enhanced.

The present leg prosthesis includes attachment means, preferably cooperative nut, bolt, and/or clamp members, to retain the various components in the desired assembled configuration. A somewhat related embodiment of my invention includes a mating curvilinear surface formed by material affixed or bonded to the relatively flat attachment surface or portion.

Certainly, for prosthetic devices which include attachment sections with surfaces which are neither flat nor of correspondingly mating configuration with respect to the curved exterior of the above-described cylindrical tube, an embodiment may be provided in a configuration which will facilitate the desired mating between the pylon and the other prosthetic device. Consequently, prosthetists and wearers can select from a broad range of prosthetic devices and readily attach same to the pylon at their discretion. Through the provision of preferably demountable attachment means such as nut and bolt assemblies or gripping clamps for attaching the prosthetic device to the pylon, the pylon may be provided in standard lengths but still be readily "custom-fitted" to a wide range of effective lengths.

Other objects and advantages of the invention will be apparent from the following specification and the accompanying drawings, which are for the purpose of illustration only.
FIG. 1 is a side elevation, partly sectional view of a prosthesis illustrating a known method of attaching a prosthetic foot below a tubular cylindrical pylon;
FIG. 2 is a perspective view of the preferred embodiment of an attachment construction in accordance with the teachings of the invention;
FIG. 3 is a partially sectional view, taken along line 3-3 of FIG. 2;
FIG. 4 is a partially sectional plan view, taken along line 4-4 of FIG. 3;
FIG. 5 illustrates an alternative embodiment of attachment means for a construction otherwise similar to FIG. 2;
FIG. 6 is a perspective view of yet another alternative embodiment of an attachment construction in accordance with the teachings of the invention;
FIG. 7 is a partially sectional view, taken along line 7-7 of FIG. 6; and
FIG. 8 is a partially sectional plan view, taken along line 8-8 of FIG. 7.

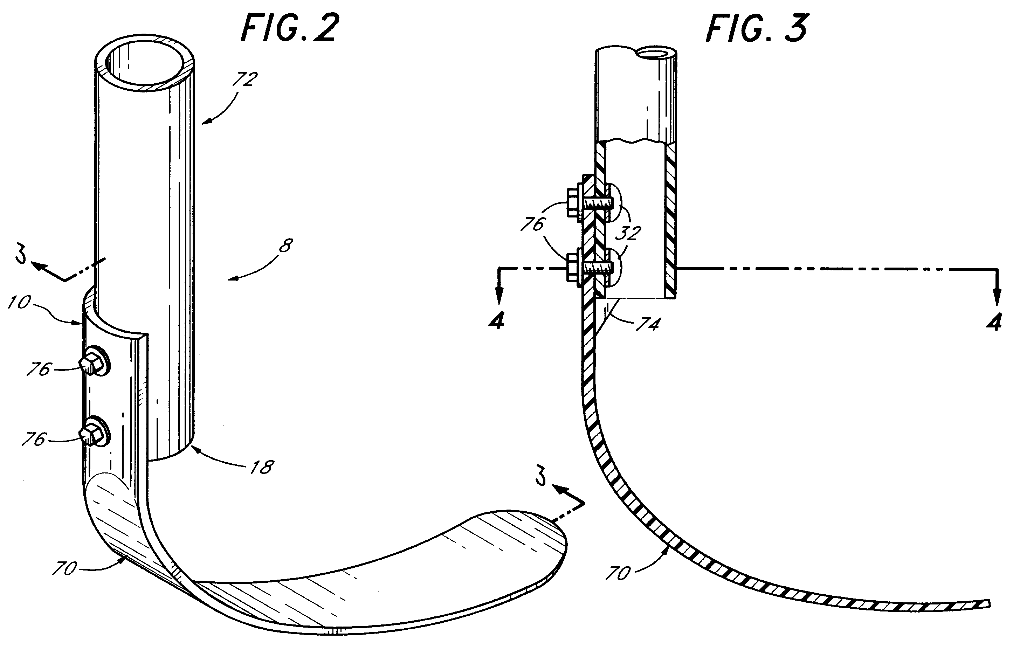

Referring to the drawings, I show a prosthesis attachment construction assembled in accordance with embodiments of my invention as shown in Figures 2-8. The pylon 72 is illustrated as having a cylindrical tubular configuration; those skilled in the art will understand that other pylon configurations may benefit from the advantages of my invention.

For purposes of illustration, the prosthesis 8 is shown as including a prosthetic foot 70 which incorporates an attachment section 10 having an attachment surface 12 at an upper end thereof for operatively attaching the prosthetic foot to the pylon 72. The upper end of the pylon 72 is intended to be operatively secured to the leg of the amputee (not shown). Those skilled in the art will understand, however, that other prosthetic feet may be utilized with a pylon and may benefit from my invention.

In the preferred embodiment, the pylon 72 is provided at its first or upper end (not shown) with a socket or other expedient for operative attachment to the wearer. Moreover, the pylon 72 and the foot 70 are preferably fabricated from lightweight, strong materials such as graphite, fiberglass, carbon fiber or the like, and, preferably provides desirable energy-performance characteristics to enhance the wearer's use of the prosthesis.

In the preferred embodiment, the pylon 72 includes a second or lower end 18 to which another prosthesis such as the prosthetic foot 70 is to be attached. In order to provide a large contact area for mating engagement between the lower end 18 and the prosthetic foot 70, the foot is curved at the upper attachment area 10. The relatively large contact area enhances the stability of the attachment of the foot 70 to the pylon 72, and does so in a simple and economic manner.

In the preferred embodiment, the attachment portion 10 may be conveniently described as having a first surface 12 matingly corresponding to the curved external surface of the second end 18 of the pylon 72. Weight, cost and performance considerations indicate that the attachment construction 10 preferably extend only a minimum distance along the pylon 72.

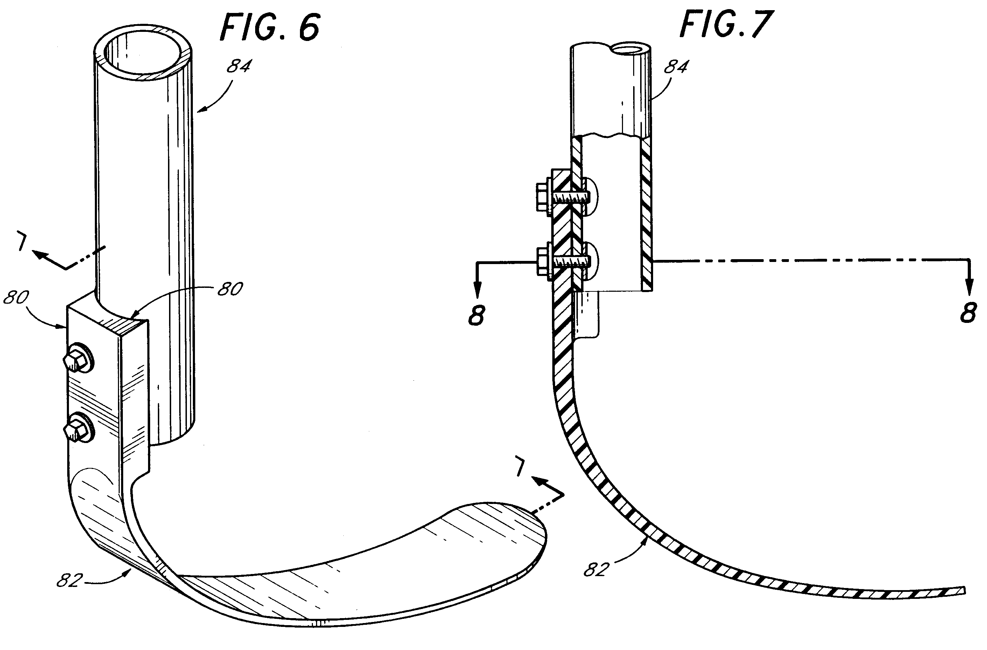

As shown in Fig. 6, alternative embodiments would include provision of an attachment portion 80 of a thickness that would space the prosthetic foot member 82 relatively further from the pylon 84, but such spacing could require additional materials and would additionally offset the prosthetic foot member 82 from the longitudinal axis of the pylon 84, with potentially undesirable performance effects.

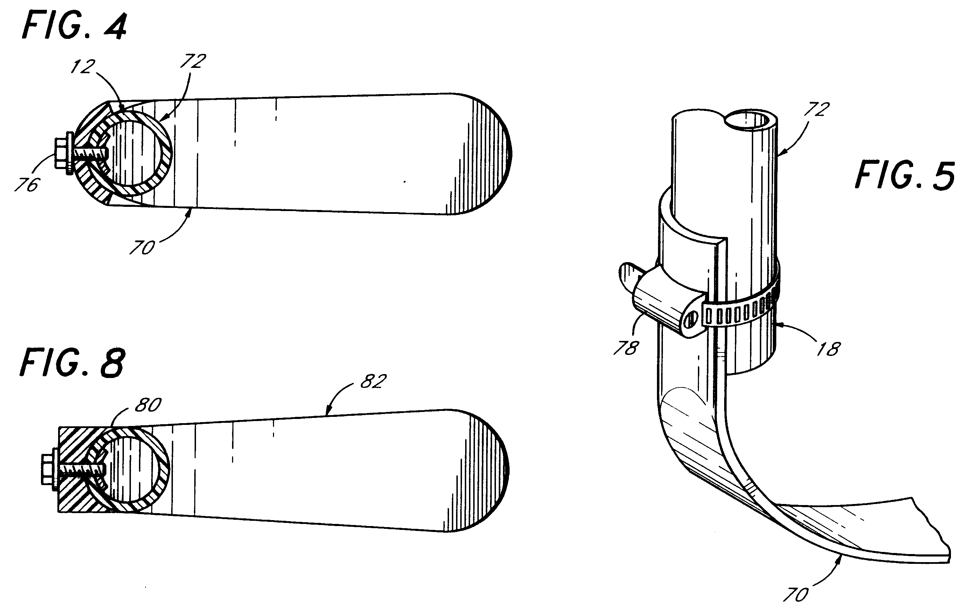

In addition, a preferred embodiment includes attachment means such as nut and bolt combinations 76. The attachment means preferably retains the pylon 72, and the lower leg prosthesis 70 in operative relationship with each other. An alternative attachment means include hose clamps or other frictionally-gripping clamp, such as those shown in Figure 5, which would function by encircling the pylon end 18.

The preferred nuts 32 are preferably threaded washers (sometimes called barrel nuts) which are curved, as best illustrated in FIG. 3 and 4, to permit their ready juxtaposition to the interior of the tubular pylon 72. In order to provide ease of assembly, the barrel nuts 32 are preferably affixed or bonded to the interior of the pylon 72. For durability and resistance to rust or other deterioration, the nuts 32 are preferably fabricated from stainless steel, titanium, or similar material.

The preferred embodiment of my invention is assembled by inserting bolts 76 through openings in the prosthetic foot member 70 and the pylon 72 and into threaded engagement with the barrel nuts 32. Another alternative embodiment would obviate the need for the barrel nuts 32. One such embodiment would involve providing threaded holes in the pylon 72, and the bolts 76 threadedly engaging only those holes instead of the barrel nuts 32.

Additionally, among the alternative embodiments of the pylon tube 72 is that the lower end 18 can be open or closed on the bottom. If barrel nuts 32 are to be installed inside the pylon 72 to provide the aforesaid attachment means, such installation would occur prior to closure of the bottom of the tube.

As will be readily understood by those skilled in the art, the attachment construction 10 may be located on either the front (not shown) or back side of the pylon 72. In any of these orientations, the attachment construction 10 provides an interface attachment surface to which a variety of prostheses may be attached.

The desired mating interface of the lower prosthesis 70 and the pylon 72 is provided by shaping the upper end 10 of prosthesis 70 into a mating configuration. This is preferably accomplished by forming the desired curvature in the attachment portion 10 of the prosthesis 70. When the prosthesis 70 is fabricated from certain deformable materials (such as aluminium or the like), however, the curvature may be provided during subsequent fabrication steps, such as by stamping the curvature into the prosthesis 70.

By limiting the downward extension of the attachment surface 10, such as indicated at 74 in FIG. 3, the attachment surface will cause a minimum of interference with the flexural performance of the prosthesis 70. Although a nut, bolt and barrel washer combination 76 is illustrated in FIGS. 2-4 for attaching the components of this embodiment, many alternative attachment means may be utilized.

The embodiment of FIGS. 6-8 is similar to that of FIGS. 2-5, but illustrates an alternative method of forming the curvilinear attachment interface 80 on the upper end of the prosthesis 82. In this embodiment, an attachment portion 80 is preferably formed during manufacture or fabrication of the prosthesis 82, and is shaped to matingly engage the exterior of the pylon 84. Alternative methods of manufacture would include depositing or affixing material (such as graphite or the like) on the prosthesis 82 after the prosthesis has been manufactured.

Similar to the embodiment of FIGS. 2-5, the components of FIGS. 6-8 may be operatively retained in their desired assembled form by any expedient means, such as, for example, nut, bolt and barrel washer combinations.

The prosthetic construction provides a simple construction which permits the ready and economic use of a popular cylindrical tubular pylon with any of a broad range of other prosthetic devices. The construction permits ready interchangeability and manufacture of the pylon and the other prosthetic devices.

Moreover, the various components and portions of the present invention may be provided in a variety of sizes, thicknesses, and materials which may be interchangeable with correspondingly-shaped components to permit fine-tuning of the prosthesis to the needs of the wearer thereof. The pylon assembly of my invention may also be used in combination with numerous prior art prosthetic devices to improve the performance of such prior art devices.

The prosthetic construction of my invention has been described with some particularity but the specific designs and constructions disclosed are not to be taken as delimiting of the invention in that various obvious modifications will at once make themselves apparent to those of ordinary skill in the art, all of which are intended to be encompassed within the appended claims.

## Claims

1. A leg prosthesis comprising the combination of a cylindrical tubular pylon (72, 84) having a front and a back side and a prosthetic foot (70, 82), said pylon (72, 84) being sized and configured so as to extend from a first end attachable to a wearer's stump to a second end (18) for securement to said prosthetic foot (70, 82), wherein the prosthetic foot (70, 82) has a relatively flat surface and includes an attachment portion (10, 80) having an attachment surface which is of a configuration matingly corresponding to the curved external surface of the tubular pylon (72, 84), said attachment surface being in direct contact with said curved external surface, and wherein said attachment portion (10, 80) is located on either the front or back side of said second end (18) of said tubular pylon (72, 84), when mounted, said leg prosthesis further comprising attachment means which retain the tubular pylon and the prosthetic foot in operative relationship with each other.

2. The leg prosthesis of Claim 1, wherein said attachment surface is formed by material affixed or bonded to said attachment portion (80) whereby said prosthetic foot (82) is spaced relatively farther from said pylon (84).

3. The leg prosthesis of Claim 1 or 2, wherein the attachment portion (10, 80) is located on the front side of said pylon (72, 84), when mounted.

4. The leg prosthesis of Claim 1 or 2, wherein the attachment portion (10, 80) is located on the back side of said pylon (72, 84), when mounted.

5. The leg prosthesis of any one of the Claims 1 to 4, wherein the prosthetic foot (70, 82) is demountably attached to the tubular pylon (72, 84) by said attachment means comprising bolts (76) inserted through openings in the prosthetic foot (70, 82) and the pylon (72, 84) and in threaded engagement with barrel nuts (32) disposed within the tubular pylon.

6. The leg prosthesis of Claim 5, wherein said barrel nuts (32) are affixed or bonded to the interior of said pylon (72).

7. The leg prosthesis of Claim 5 or 6, wherein said barrel nuts (32) are fabricated from steel or titanium.

8. The leg prosthesis of any one of the Claims 1 to 4, wherein the prosthetic foot (70, 82) is demountably attached to the tubular pylon (72, 84) by said attachment means comprising bolts (76) inserted through openings in the prosthetic foot (70, 82) and corresponding threaded openings in the pylon (72, 84).

## Patentansprüche

1. Unterschenkelprothese aufweisend eine Kombination einer zylindrischen Rohrstütze (72, 84) mit einer Vorderseite und einer Rückseite und einer Fußprothese (70, 82), wobei: die Stütze (72, 84) so bemessen und konfiguriert ist, daß sie sich von einem an dem Stumpf des Trägers befestigbaren ersten Ende zu einem Zweiten Ende (18) für die Befestigung der Fußprothese (70, 82) erstreckt; die Fußprothese (70, 82) eine relativ flache Oberfläche aufweist und einen Befestigungsabschnitt (10, 80) mit einer Befestigungsoberfläche enthält, welche eine Konfiguration besitzt, die der gekrümmten Außenoberfläche des Stützrohres (72, 84) paarig entspricht; die Befestigungsoberfläche in direktem Kontakt mit der gekrümmten Außenoberfläche steht; und der Befestigungsabschnitt (10, 80) nach der Montage entweder an der Vorder- oder der Rückseite des zweiten Endes (18) des Rohrstütze (21, 84) angeordnet ist und die Fußprothese ferner eine Befestigungseinrichtung aufweist, welche die Rohrstütze und die Fußprothese in einer Betriebsbeziehung zueinander hält.

2. Unterschenkelprothese nach Anspruch 1, wobei die Befestigungsoberfläche aus einem an dem Befestigungsabschnitt (80) befestigten oder damit verklebten Material ausgebildet ist, wodurch die Fußprothese (82) von der Stütze (84) relativ weiter entfernt ist.

3. Unterschenkelprothese nach Anspruch 1 oder 2, wobei der Befestigungsabschnitt (10, 80) nach der Montage an der Vorderseite der Stütze (72, 84) angeordnet ist.

4. Unterschenkelprothese nach Anspruch 1 oder 2, wobei der Befestigungsabschnitt (10, 80) nach der Montage an der Rückseite der Stütze (72, 84) angeordnet ist.

5. Unterschenkelprothese nach einem der Ansprüche 1 bis 4, wobei die Fußprothese (70, 82) abnehmbar an der Rohrstütze (70, 84) durch die Befestigungseinrichtung befestigt ist, welche aus Schrauben besteht, die durch Öffnungen in der Fußprothese (70, 82) und der Stütze (72, 81) hindurchgesteckt sind und mit im Inneren der Rohrstütze angeordneten Spannschloßmuttern (32) in einem Gewindeeingriff stehen.

6. Unterschenkelprothese nach Anspruch 5, wobei die Spannschloßmuttern (32) an der Innenseite der Stütze (72) befestigt oder damit verklebt sind.

7. Unterschenkelprothese nach Anspruch 5 oder 6, wobei die Spannschloßmuttern (32) aus Stahl oder Titan hergestellt sind.

8. Unterschenkelprothese nach Anspruch 1 bis 4, wobei die Fußprothese (70, 82) abnehmbar an der Rohrstütze (72, 84) durch die Befestigungseinrichtung befestigt ist, welche aus Schrauben (76) besteht, die durch Öffnungen in der Fußprothese (70, 82) und entsprechende Gewindeöffnungen (72, 84) in der Stütze (72, 84) hindurch eingeführt sind.

## Revendications

1. Prothèse de jambe comprenant la combinaison d'un montant tubulaire (72,84) cylindrique ayant un côté avant et un côté arrière et un pied prothétique (70,82), ledit montant (72,84) étant dimensionné et configuré de façon à s'étendre depuis une première extrémité pouvant être réunie au moignon d'un porteur à une seconde extrémité (18) pour la fixation audit pied prothétique (70,82), dans laquelle le pied prothétique (70,82) a une surface relativement plate et comprend une portion de réunion (10,80) ayant une surface de réunion qui a une configuration correspondant de façon appariée à la surface externe courbe du montant tubulaire (72,84), ladite surface de réunion étant en contact direct avec ladite surface extérieure courbe, et dans laquelle ladite portion de réunion (10,80) est située sur le côté avant ou arrière de ladite seconde extrémité (18) dudit montant tubulaire (72,84), a l'état monté, ladite prothèse de jambe comprenant en outre des moyens de réunion qui retiennent le montant tubulaire et le pied prothétique en relation opérationnelle l'un avec l'autre.

2. Prothèse de jambe selon la revendication 1, dans laquelle ladite surface de réunion est formée par un matériau solidarisé ou lié à ladite portion de réunion (80), grâce à quoi ledit pied prothétique (82) est relativement plus éloigné dudit montant (84).

3. Prothèse de jambe selon la revendication 1 ou 2, dans laquelle ladite portion de réunion (10,80) est située sur le côté avant dudit montant (72,84), à l'état monté.

4. Prothèse de jambe selon la revendication 1 ou 2, dans laquelle ladite portion de réunion (10,80) est située sur le côté arrière dudit montant (72,84), à l'état monté.

5. Prothèse de jambe selon l'une quelconque des revendications 1 à 4, dans laquelle le pied prothétique (70,82) est réuni de façon démontable au montant tubulaire (72,84) par lesdits moyens de réunion comprenant des boulons (76) insérés au travers d'ouvertures ménagées dans le pied prothétique (70,82) et le montant (72,84) et vissés dans des écrous barillets (32) disposés au sein du montant tubulaire.

6. Prothèse de jambe selon la revendication 5, dans laquelle lesdits écrous barillets (32) sont fixés ou liés à l'intérieur dudit montant (72).

7. Prothèse de jambe selon la revendication 5 ou 6, dans laquelle lesdits écrous barillets (32) sont fabriqués en acier ou en titane.

8. Prothèse de jambe selon l'une quelconque des revendications 1 à 4, dans laquelle le pied prothétique (70,82) est réuni de façon démontable au montant tubulaire (72,84) par lesdits moyens de réunion comprenant des boulons (76) insérés au travers d'ouvertures ménagées dans le pied prothétique (70,82) et des ouvertures correspondantes filetées ménagées dans le montant (72,84).
